(19) Europäisches Patentamt / European Patent Office / Office européen des brevets

(11) EP 4 635 941 A1

(12) EUROPEAN PATENT APPLICATION

(43) Date of publication:
**22.10.2025 Bulletin 2025/43**

(21) Application number: **25172444.9**

(22) Date of filing: **14.05.2024**

(51) International Patent Classification (IPC):
***C07C 409/38** (2006.01)* ***C07C 407/00** (2006.01)*

(52) Cooperative Patent Classification (CPC):
(C-Sets available)
**C07C 407/00** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **01.06.2023 EP 23176680**

(62) Document number(s) of the earlier application(s) in accordance with Art. 76 EPC:
**24175823.4 / 4 471 010**

(71) Applicant: **Nouryon Chemicals International B.V.**
**1101 BZ Amsterdam (NL)**

(72) Inventors:
- **BEEK, Waldo Joseph Elisabeth**
  **1101 BZ Amsterdam (NL)**
- **TER HORST, Bjorn**
  **1101 BZ Amsterdam (NL)**

(74) Representative: **Ingrassia, Fisher & Lorenz UK Ltd.**
**Cambridge House**
**Henry Street**
**Bath BA1 1BT (GB)**

Remarks:
This application was filed on 24-04-2025 as a divisional application to the application mentioned under INID code 62.

# (54) PROCESS FOR PREPARING A PEROXYESTER OR PEROXYCARBONATE

(57) The present disclosure relates to a process for preparing a peroxyester or peroxycarbonate comprising:
a) reacting tert-butyl hydroperoxide with an acid halide, an acid anhydride, or a haloformate, in the presence of a base,
b) separating the aqueous layer after completion of step a),
c) adding a reducing agent to the organic layer after the aqueous layer has been separated in step b), wherein the reducing agent is an agent capable of reducing tert-butyl hydroperoxide to the tert-butyl alcohol,
d) ensuring that the mixture of step c) has a pH of greater than 6.8 by maintaining or increasing the pH of the mixture of step c), and
e) maintaining the pH of greater than 6.8 for at least 5 seconds,
wherein the process is characterized in that:
i) the mixture of step c) has a pH of less than 6.8 before step d), and in step d) the pH of the mixture of step c) is increased to a pH of greater than 6.8; and/or
ii) after completion of step e) the pH is decreased to a pH of less than 6.8.

EP 4 635 941 A1

(52) Cooperative Patent Classification (CPC): (Cont.)

C-Sets
**C07C 407/00, C07C 409/38**

## Description

### Technical Field

The present disclosure relates to a process for preparing a peroxyester or peroxycarbonate with improved storage stability and reduced hydroperoxide content.

### Background

Peroxyesters and percarbonates find a wide variety of uses in the chemical field. They are typically produced by reacting an organic hydroperoxide with a reactive carbonyl species, such as an acid halide, an acid anhydride, or a haloformate.

An issue with peroxyesters and percarbonates is that they often contain undesirably high levels of hydroperoxide, which can be problematic from a regulatory and/or application viewpoint. Additionally, peroxyesters and percarbonates often have poor storage stability.

The standard workup process for this reaction comprises one or more washing steps in an attempt to reduce the residual organic hydroperoxide present in the organic layer (e.g., EP1382596). In CN112300044 the reaction workup was performed at pH 7.5-10, which was found by the present inventors to yield a poor product (see Comparative Example 5 below). The present inventors unexpectedly found that the hydroperoxide content in the peroxide product could be substantially reduced whilst simultaneously giving rise to a peroxy product with much improved storage stability by a specific process that manipulates the reaction mixture pH during the initial workup process.

### Description

In a first aspect, the present disclosure relates to a process for preparing a peroxyester or peroxycarbonate comprising:

a) reacting an organic hydroperoxide with an acid halide, an acid anhydride, or a haloformate, in the presence of a base,
b) separating the aqueous layer after completion of step a),
c) adding a reducing agent to the organic layer after the aqueous layer has been separated in step b), wherein the reducing agent is an agent capable of reducing the organic hydroperoxide to the corresponding alcohol,
d) ensuring that the mixture of step c) has a pH of greater than 6.8, preferably greater than 7.0, preferably greater than 7.2, and more preferably greater than 7.4, by maintaining or increasing the pH of the mixture of step c), and
e) maintaining the pH of greater than 6.8, preferably greater than 7.0, preferably greater than 7.2, and more preferably greater than 7.4, for at least 5 seconds, preferably at least 10 seconds, preferably at least 30 seconds, preferably at least 60 seconds, and more preferably at least 120 seconds,

wherein the process is characterized in that:

i. the mixture of step c) has a pH of less than 6.8 before step d), preferably a pH of about 6.5 or lower, and most preferably a pH within the range of about 4 to 6.5, and in step d) the pH of the mixture of step c) is increased to a pH of greater than 6.8, preferably greater than 7.0, preferably greater than 7.2, and more preferably greater than 7.4; and/or
ii. after completion of step e) the pH is decreased to a pH of less than 6.8, preferably to a pH of about 6.5 or lower, and most preferably to a pH within the range of about 4 to 6.5.

Steps a) and b) follow the normal process for producing peroxyesters or peroxycarbonates, wherein the organic hydroperoxide is reacted with an acid halide, an acid anhydride, or a haloformate, in the presence of a base. The equivalent amount of the reactive carbonyl compound (acid halide, acid anhydride, or haloformate) relative to the organic hydroperoxide is not particularly limited, but typically is in the range of about 0.25-5 equivalents, preferably in the range of about 0.6-1.1 equivalents, more preferably in the range of about 0.6-1 equivalents, and most preferably in the range of about 0.8 - 1 equivalents.

The reaction conditions of step a) are conventional. The temperature generally is in the range of -10 °C to 70°C and suitably between 0 °C to 50 °C. The pH is basic, i.e., above 7. Generally, the pH is in the range of 9-14. In practice, the pH is above 10, and the common pH range is from 11 to 13.5. The reaction may proceed under ambient pressure and in free contact with the atmosphere. Any suitable base may be used to adjust the pH to a basic pH, such as, but not limited to, sodium carbonate, potassium carbonate, sodium hydroxide, potassium hydroxide, and mixtures thereof. Such bases are typically used in the form of aqueous solutions thereof. The reaction mixture may also comprise conventional auxiliaries,

such as NaCl(aq.) to assist phase separation (for step b)). The reaction can be performed neat or in the presence of solvent(s).

The organic hydroperoxide may be selected from an organic hydroperoxide of the general formula (II):

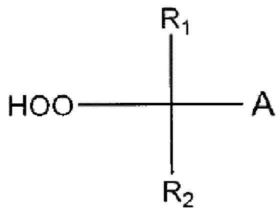

(II)

wherein:

**$R_1$** and **$R_2$** are independently selected from the group comprising hydrogen, $C_1$-$C_{20}$ alkyl, $C_3$-$C_{20}$ cycloalkyl, $C_6$-$C_{20}$ aryl, $C_7$-$C_{20}$ aralkyl, and $C_7$-$C_{20}$ alkaryl, or **$R_1$** and **$R_2$** form a $C_3$-$C_{12}$ cycloakyl group, which groups may include linear or branched alkyl moieties, and

each of **$R_1$** and **$R_2$** may optionally be substituted with one or more groups selected from hydroxy, hydroperoxy, alkoxy, linear or branched alkyl, aryloxy, halogen, ester, carboxy, nitrile, and amido,

**A** is selected independently of $R_1$ and $R_2$ from the same group of substituents as $R_1$ and $R_2$, or **A** is of the general formula (III):

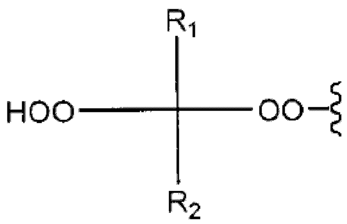

(III)

wherein **$R_1$** and **$R_2$** are as defined above, wherein **A** is preferably selected independently of $R_1$ and $R_2$ from the same group of substituents as $R_1$ and $R_2$.

Preferred organic hydroperoxides include tert-butyl hydroperoxide, tert-amyl hydroperoxide, cumyl hydroperoxide, 1,1,3,3-tetramethylbutyl hydroperoxide, 4-hydroperoxy-4-methylpentan-2-ol, 2,5-dihydroperoxy-2,5-dimethylhex-3-yne, 2,5-dihydroperoxy-2,5-dimethylhexane, or mixtures thereof. The most preferred organic hydroperoxide for use in this process is tert-butyl hydroperoxide (TBHP), preferably tert-butyl hydroperoxide obtained from an air oxidation process.

The acid halide, acid anhydride, or haloformate may be a reactive carbonyl compound of the general formulae (Ia) or (Ib) :

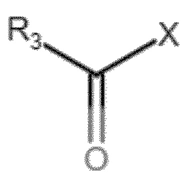

(Ia)

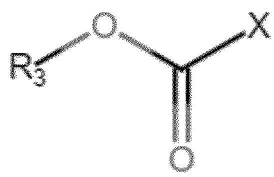

(Ib)

wherein:

**$R_3$** is independently selected from the group comprising $C_1$-$C_{20}$ alkyl, $C_3$-$C_{20}$ cycloalkyl, $C_6$-$C_{20}$ aryl, $C_7$-$C_{20}$ aralkyl, and $C_7$-$C_{20}$ alkaryl, wherein $R_3$ is optionally substituted with one or more groups selected from hydroxy, alkoxy, linear or

branched alkyl, aryloxy, halogen, ester, carboxy, nitrile, and amido, and

**X** is a halide (preferably chloride) or -O-CO-$R_{3'}$, wherein $R_{3'}$ is selected independently of $R_3$ from the same group of substituents as $R_3$.

Preferably, the acid halide or acid anhydride is derived from any of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid, citraconic acid, fumaric acid, oxalic acid, terephthalic acid, propenoic acid, and phthalic acid.

Preferably, the process uses an acid halide or a haloformate.

Preferably, the acid halide is an acid chloride. Preferably, the acyl portion of the acid chloride corresponds to the acyl portion of any of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid, citraconic acid, fumaric acid, oxalic acid, terephthalic acid, propenoic acid, and phthalic acid.

Preferably, the haloformate is a chloroformate. Preferred haloformates include 2-(1-methylethoxy)phenyl chloroformate, 1-methylpropyl chloroformate, 4-methylphenyl chloroformate, heptyl chloroformate, cyclohexyl methyl chloroformate, ethylene glycol bis(chloroformate), phenyl chloroformate, 3-methoxybutyl chloroformate, 2-phenoxyethyl chloroformate, 2,2-dimethyl-1,3-propane diol bis(chloroformate), phenyl methyl chloroformate, 9-octadecenyl chloroformate, 2-methylphenyl chloroformate, bisphenol A bis(chloroformate), 1,3-dimethyl butyl chloroformate, 3,4-dimethyl butyl chloroformate, 3,4-dimethyl phenyl chloroformate, 1,4-butane diol bis(chloroformate), 1,1-bis (ethoxycarbo)ethyl chloroformate, 3,5-dimethyl phenyl chloroformate, octyl chloroformate, ethyl chloroformate, octadecyl chloroformate, (2-oxo-1,3-dioxolan-4-yl)methyl chloroformate, 1,6-hexane diol bis(chloroformate), 2-chlorobutyl chloroformate, 4-methoxyphenyl chloroformate, 2-methylpropyl chloroformate, , dodecyl chloroformate, 1,4-cyclohexane dimethanol bis(chloroformate), 2-chloro-2-phenyl ethyl chloroformate, 2-acryloyloxyethyl chloroformate, 4-nitrophenyl chloroformate, n-butyl chloroformate, decyl chloroformate, 2-ethylhexyl chloroformate, 2-propenyl chloroformate, 2-chlorocyclohexyl chloroformate, 2-methyl-2-propenyl chloroformate, cyclohexyl chloroformate, 2-chloroethyl chloroformate, [4-(phenylazo)phenyl]methyl chloroformate, hexadecyl chloroformate, 1-naphthalenyl chloroformate, chloroformate, 3,5,5-trimethyl hexyl chloroformate, isotridecyl chloroformate, tridecyl chloroformate, 4-(1,1-dimethylethyl)cyclohexyl chloroformate, chloroformate, 3-chloropropyl chloroformate, tetradecyl chloroformate, chloroformate, methyl chloroformate, 2-(1-methylethyl) phenyl chloroformate, triethylene glycol bis(chloroformate), 2-methoxyethyl chloroformate, 1-methylethenyl chloroformate, 3-methylphenyl chloroformate, 2 chloroformate, diethylene glycol bis(chloroformate), 3-methyl-5-(1-methylethyl) phenyl chloroformate, , 2-ethoxyethyl chloroformate, 3-methyl-1,5-pentane diol bis(chloroformate), 4-methoxy carbophenyl chloroformate, ethenyl chloroformate, 1-methylethyl chloroformate, 2-(1-methylpropyl)phenyl chloroformate, chloroformate, pentyl chloroformate, cyclodecyl chloroformate, 4-(1,1-dimethylethyl)phenyl chloroformate, hexyl chloroformate, n-propyl chloroformate, 3-methoxy-3-methylbutyl chloroformate, 2-propoxyethyl chloroformate, 2-methoxy-1-methylethyl chloroformate, 2-butoxyethyl chloroformate, 2,2-dimethyl propyl chloroformate, 2,3-dihydro-2,2-dimethyl-7-benzofuranyl chloroformate, 1-chloroethyl chloroformate, cyclobutyl chloroformate, 5-methyl-2-(1-methylethyl)cyclohexyl chloroformate, 1,1-dimethyl ethyl chloroformate, 1-methylheptyl chloroformate, and mixtures thereof.

After completion of step a), the reaction mixture is allowed to settle, thereby forming an aqueous layer and an organic layer (i.e., two separate/immiscible phases). The aqueous layer that forms upon allowing the mixture to settle is separated in step b). The organic layer that remains is taken through to step c).

In step c), the reducing agent is added to the organic layer. Preferably, the reducing agent is a sulfur based reducing agent, such as a dithionite, hydrosulfite, metabisulfite, sulfide or a sulfite. The reducing agent serves to reduce any remaining hydroperoxide to the corresponding alcohol. Preferred sulfites include metal sulfites, metal bisulfites, and/or metal metabisulfites. Preferred metals include the alkali and alkaline metals, such as sodium or potassium. Preferably, the reducing agent added in step c) is sodium metabisulphite and/or sodium sulfite, preferably sodium metabisulphite. The reducing agent is preferably added in step c) as an aqueous solution of the reducing agent. As such, in a most preferred embodiment, the reducing agent added in step c) is an aqueous solution of sodium metabisulphite. The reducing agent (RA) to hydroperoxide (HP) molar ratio (RA:HP) is preferably >1:1, preferably >1:1 to about 5:1, preferably >1:1 to about 3:1, more preferably >1:1 to about 2:1, and most preferably about 1.3:1 to about 1.8:1. It is preferred if the reaction mixture is agitated (e.g., stirred) for at least about 1 minute (e.g., about 1-10 minutes) before proceeding to the next step of the process.

In a preferred embodiment (*step i)*), the pH of the mixture of step c) is lowered to a pH of less than 6.8 before proceeding to step d), preferably to a pH of about 6.5 or lower, and most preferably to a pH within the range of about 4 to 6.5.

The pH drop may be achieved by any conventional means, such as by adding an acid, e.g., $H_2SO_4$(aq.), by adding a buffer with a pH of less than 6.8, and/or by using a reducing agent with a pH of less than 6.8. The pH of the mixture of step c) may be decreased as a consequence of dosing the reducing agent to the organic layer (preferred reducing agents, such as aqueous sulfite solutions, can be weakly acidic, so dosing those acidic reducing agents to the organic layer in step c) may result in the mixture having a pH of <6.8). Preferably, a buffer with a pH in the range of about pH 4 - 6.5 is used to effect the pH drop. Preferred buffers include acetate buffer with a pH of from about 4 to 6.5 (other suitable buffers include, but are not limited to, citrate acid buffer or monopotassiumphosphate buffer). The mixture is preferably agitated (e.g., stirred) at this reduced pH for at least about 1 minute (e.g., about 1 to about 25 minutes) before proceeding to the next step of the process.

Step d) ensures that the pH of the mixture of step c) is at a pH of greater than 6.8, preferably greater than 7.0, preferably greater than 7.2, and more preferably greater than 7.4. For example, the pH may be >8. This is achieved either by maintaining the pH of the mixture (if the mixture of step c) is already at a pH of >6.8) or by increasing the pH of the mixture to a pH of greater than 6.8 (particularly if the pH of the mixture of step c) is adjusted to a pH of less than 6.8 before step d)). A pH increase may be achieved by adding a base in an amount necessary to reach the desired pH value. Suitable bases include, but are not limited to, sodium carbonate, potassium carbonate, sodium hydroxide, potassium hydroxide, and mixtures thereof. Such bases are typically used in the form of aqueous solutions thereof.

In step e), the pH of greater than 6.8, preferably greater than 7.0, preferably greater than 7.2, and more preferably greater than 7.4, is maintained for at least 5 seconds, preferably at least 10 seconds, preferably at least 30 seconds, preferably at least 60 seconds, and more preferably at least 120 seconds, such as from about 120 seconds to about 600 seconds. The mixture is preferably agitated (e.g., stirred) during step e).

In a preferred embodiment, the process further comprises step f) (step f) = *step ii)*), wherein after completion of step e) the pH of the mixture is subsequently decreased to a pH of less than 6.8, preferably to a pH of about 6.5 or lower, and most preferably to a pH within the range of about 4 to 6.5. The pH drop may be achieved by any conventional means, such as by adding an acid, e.g., $H_2SO_4$(aq.) or by adding a buffer with a pH of less than 6.8. Preferably, a buffer with a pH in the range of about pH 4 - 6.5 is used to effect the pH drop. Preferred buffers include acetate buffer with a pH of from about 4 to 6.5 (other suitable buffers include, but are not limited to, citrate acid buffer or monopotassium phosphate buffer). The pH of the mixture of step f) is preferably maintained for at least 5 seconds, preferably at least 10 seconds, preferably at least 30 seconds, preferably at least 60 seconds, such as from about 90 seconds to about 600 seconds, during which time the mixture is preferably agitated (e.g., stirred). Optionally, at the end of step f) the pH may be increased to a pH of greater than 6.8 (i.e., *step d*) may be optionally repeated at the end of *step f)*).

The aqueous layer of step f) (that forms after the mixture is allowed to settle into an aqueous layer and an organic layer, i.e., two immiscible phases) is separated, and the remaining organic layer may be subjected to one or more washing steps g) (to remove any residual water-soluble impurities, such as excess sulfite, alcohol and sulfate generated during the reaction). The washing step(s) may comprise one or more water washes and/or one or more alkaline aqueous washes (e.g., washing with NaOH(aq) and/or $NaHCO_3$(aq)). The washing step(s) preferably comprises one or more neutral to alkaline aqueous washes. For the avoidance of doubt, the peroxyester or peroxycarbonate is present in this organic layer. The final organic layer may be dried using conventional means, such as drying agents (e.g., $MgSO_4$), vacuum, and/or air drying.

It is preferable for the temperature of the mixture during steps c)-g) to be maintained at about 0-70°C, more preferably about 0-40°C.

In a preferred embodiment, the present disclosure relates to a process for preparing a peroxyester or peroxycarbonate comprising:

a) reacting an organic hydroperoxide with an acid halide, an acid anhydride, or a haloformate, in the presence of a base,

b) separating the aqueous layer after completion of step a),

c) adding a reducing agent to the organic layer after the aqueous layer has been separated in step b), wherein the reducing agent is an agent capable of reducing the organic hydroperoxide to the corresponding alcohol, and wherein the mixture of the organic layer and the reducing agent has a pH of less than 6.8, preferably a pH of about 6.5 or lower, and most preferably a pH within the range of about 4 to 6.5,

d) increasing the pH of the mixture of step c) to a pH of greater than 6.8, preferably greater than 7.0, preferably greater than 7.2, and more preferably greater than 7.4,

e) maintaining the pH of greater than 6.8, preferably greater than 7.0, preferably greater than 7.2, and more preferably greater than 7.4, for at least 5 seconds, preferably at least 10 seconds, preferably at least 30 seconds, preferably at least 60 seconds, and more preferably at least 120 seconds,

f) decreasing the pH after completion of step e) to a pH of less than 6.8, preferably to a pH of about 6.5 or lower, and most preferably to a pH within the range of about 4 to 6.5, and

g) optionally repeating step d) (i.e., increase the pH of the mixture of step f) to a pH or greater than 6.8), and/or optionally washing the organic layer of step f), wherein the optional washing step(s) preferably comprise one or more

water washes and/or one or more alkaline aqueous washes (e.g., washing with NaOH(aq) and/or $NaHCO_3$(aq)).

This process is particularly relevant for processes which use hydroperoxides manufactured by air oxidation (notably t-butyl hydroperoxide). In that respect, a surprising observation was that, using the preferred processes disclosed herein, it was possible to consistently produce peroxyesters and peroxycarbonates from t-butyl hydroperoxide that have an initial (t = 0 weeks) tert-butylhydroperoxide (TBHP) content of less than 300 ppm *and* a four-week stability (0-4 week ΔTBHP) value of 300 ppm or less (the initial TBHP content and the four-week stability value are determined in accordance with the "TBHP Protocol" described in the Worked Examples below). This very high product purity and stability profile was unexpected.

Accordingly, in another aspect, the present disclosure relates to a tert-butyl peroxyester or a tert-butyl peroxycarbonate, preferably a tert-butyl peroxyester, characterized in that the tert-butyl peroxyester or tert-butyl peroxycarbonate has an initial (t = 0 weeks) tert-butylhydroperoxide (TBHP) content of less than 300 ppm and a four-week stability (0-4 week ΔTBHP) value of 300 ppm or less, wherein the initial TBHP content and the four-week stability value are determined in accordance with the "TBHP Protocol" as set out in the worked examples below. For the avoidance of doubt a "tert-butyl peroxyester" is a peroxyester that is obtainable by reacting TBHP with an acid halide or an acid anhydride (i.e., t-Bu-OO-C(O)R), and a "tert-butyl peroxycarbonate" is a peroxycarbonate that is obtainable by reacting TBHP with a haloformate (i.e., t-Bu-OO-C(O)OR). Non-limiting examples of tert-butyl peroxyesters include tert-butyl peroxy-3,5,5-trimethyl hexanoate (CAS: 13122-18-4), tert-Butyl peroxy-2-ethylhexanoate (CAS: 3006-82-4), and tert-Butyl peroxybenzoate (CAS: 614-45-9).

It has also been found that this process is particularly effective for preparing peroxyesters or percarbonates that do not contain any free acid groups. Accordingly, in a preferred embodiment, the process disclosed herein is for preparing a peroxyester or peroxycarbonate, wherein the peroxyester or peroxycarbonate contains no free acid groups.

**Worked Examples**

The following examples provide a detailed method for working the invention. These worked examples are exemplary in nature and not intended to be limitative.

A. Preparation of tert-butyl peroxy-3,5,5-trimethyl hexanoate (CAS:13122-18-4) [Steps a) & b)]

To a 1L jacketed glass reactor, equipped with baffles, a pH electrode, an overhead mechanical stirrer (pitch blade agitator, size 1/3 of the diameter of the reactor) and glycol/water temperature control, 148.4g TBHP (70 wt% in water) was added. The reaction medium was cooled to 10° C under agitation (1000 rpm) and 125.8g NaOH-25 solution (25 wt% NaOH in water) was added in 20 min. After the addition, agitation was increased to 1300 rpm and 93.6 gram Isononanoyl chloride was dosed in 16 min and the temperature was allowed to rise to 25° C and maintained at that temperature. In a second addition step 93.5g isononanoyl chloride and 60.4g NaOH-25 were added simultaneously in 30 min during which the temperature was allowed to rise to 30° C and maintained at that temperature. After the second addition the reaction mixture was stirred for 9 min at 30° C and 1300 rpm. The reaction mixture was quenched with 75g demineralized water and stirred for 1 min. Agitation was stopped and the organic phase and water phase were allowed to separate. The water phase was drained from the reactor and discarded.

Reducing agent addition step [Step c)]

Agitation was restarted (1000rpm) and 5.0g buffer solution (16.3 wt% AcOH, 62.5 wt% water and 21.2 wt% NaOH-25) was added followed by the addition of 105 gram water. The temperature was set to 25° C by adjusting the jacketed temperature. The pH was adjusted to pH = 5.5 by addition of a few drops of NaOH-25. To the buffered solution 55.5 grams of a freshly prepared sulfite solution (69 wt% water, 18 wt% sodium metabisulfite $Na_2S_2O_5$ (s) and 13 wt% NaOH-25) was added dropwise over a period of 20 minutes. During the dosing a pH of 5.5 was maintained by the addition of NaOH-25 (approximately 2 grams in total).

Example 1 [*step i) - the mixture of step c) has a pH of less than 6.8 before proceeding to step d)*]

After the addition of the sulfite solution, the reaction mixture was stirred for 22 minutes at a pH of 5.5. The pH of the reaction mixture was then increased to pH 8.0 by the dropwise addition of NaOH-25 (approximately 1g over 5 minutes) *(steps d) and e)),* followed by workup (see below).

Example 2 [*step i) - the mixture of step c) has a pH of less than 6.8 before proceeding to step d); and step ii) - after completion of step e) the pH is decreased to a pH of less than 6.8*]

After the addition of the sulfite solution, the reaction mixture was stirred for 5 minutes at a pH of 5.5. The pH was then increased to pH 10 by the dropwise addition of 9.0g NaOH-25 over 2 minutes (*step d)*). The mixture was stirred for 3 minutes at pH 10 (*step e)*). The pH was then lowered back to 5.5 by the dropwise addition of 4g HCl (15 wt% HCl in water) over 10 minutes (*step ii)*). The reaction mixture was stirred for 2 minutes at a pH of 5.5. Total reduction time was 22 minutes (cf. Example 1). The pH of the reaction mixture was then increased to pH 8.0 by the dropwise addition of NaOH-25 (approximately 1g over 5 minutes), followed by workup (see below)

Workup for Examples 1 and 2:

Agitation was stopped and the organic phase and water phase were allowed to separate. The water phase was drained from the reactor and discarded. Next, 55 g NaCl-25 (25 wt% NaCl in water), 180g demineralized water and 28g $NaHCO_3$-6 (6 wt% $NaHCO_3$ in water) were added to the remaining organic layer. After the addition the mixture was stirred (1000 rpm) at 25° C for 3 minutes. Agitation was stopped and the organic phase and water phase were allowed to separate. The water phase was drained from the reactor and discarded. Subsequently the organic phase was drained and collected separately in a 500 ml Erlenmeyer flask. The organic phase was dried over 10 grams $MgSO_4.2H_2O$ for 10 minutes. The mixture was filtered over a glass filter and the clear organic peroxide product 218 gram (assay 99.4%) was collected and stored at 20° C.

TBHP content determination in final product ("***TBHP Protocol***")

The following reagents and apparatus were used in the determination of the TBHP content of the peroxide product:

**Solution A:** Sulphur dioxide solution approx. 0.005 mol/L $SO_2$ solution in methanol

**Solution B:** Methyl ethyl ketone 1000 mL with approximately 15 μL tert. Butyl hydroperoxide (70 wt%) and 50 mL water.

**Equipment:** Potentiometric titrator Metrohm combined Pt Titrode (art. 6.0431.100). Conditions of Metrohm Titrando 888 are:

- Monotone titration
- Start volume: 0.1 mL
- Volume increment: 0.07 mL
- Dosing rate: 2 mL/minute
- Minimal waiting time: 0 seconds
- Maximum waiting time: 4 seconds

The TBHP content of the final peroxide product was determined by dissolving 1g of peroxide product in 25 mL Solution B. The solution was titrated immediately with the Sulphur dioxide solution A until slightly beyond the potential jump. The measurement was performed in duplicate. Two blanks were also performed, and the average blank was calculated. The following formula was used to calculate the hydroperoxide content (TBHP)

$$Hydroperoxide\ content\ (mg/kg) = \frac{(V_1 - V_0) \cdot c \cdot Mm \cdot 1000}{m}$$

Where:

$V_1$ mL of sulphur dioxide solution to titrate the sample
$V_0$ mL of sulphur dioxide to titrate the blank
*c* molarity of the sulphur dioxide solution
*Mm* molar mass of the hydroperoxide concerned, for bi functional hydroperoxides use $\frac{Mm}{2}$.
*m* mass of sample in grams

Results:

| | TBHP (ppm) t = 0 weeks | TBHP (ppm) t = 2 weeks | TBHP (ppm) t = 4 weeks | TBHP (ppm) t = 8 weeks | ΔTBHP* (ppm) 0-4 weeks | ΔTBHP* (ppm) 0-8 weeks |
|---|---|---|---|---|---|---|
| Example 1 | 1324 | 1663 | 1696 | 1732 | 372 | 408 |
| Example 2 | 179 | 330 | 364 | 436 | 185 | 257 |
| ** A lower Δ TBHP value indicates increased storage stability* | | | | | | |

For the avoidance of any doubt, the term "four-week stability (0-4 weeks ΔTBHP) value" as used herein means the difference in TBHP, in ppm, between the TBHP value measured at week 0 (i.e., the initial TBHP value) and the TBHP value measured after a storage period of four weeks, wherein both the week 0 and week 4 values are determined in accordance with the above "TBHP protocol". For the purposes of the present disclosure, the "four-week stability (0-4 weeks ΔTBHP) value" is determined by storing the peroxide for a period of 4 weeks at 20°C.

<u>B. Preparation of tert-Butyl peroxy-2-ethylhexanoate (CAS: 3006-82-4) [Steps a) and b)]</u>

To a 1L jacketed glass reactor, equipped with baffles, a pH electrode, an overhead mechanical stirrer (pitch blade agitator, size 1/3 of the diameter of the reactor) and glycol/water temperature control, 177.3g TBHP (70 wt% in water) was added. The reaction medium was kept at 25° C under agitation (1000 rpm) and 138.3g NaOH-25 solution was added in 20 min. After the addition, agitation was increased to 1300 rpm and 204.2g 2-ethyl hexanoyl chloride and 88.4g NaOH-25 were added simultaneously over 35 minutes during which the temperature was allowed to rise to 40° C and maintained at that temperature. After the addition the reaction mixture was stirred for an additional 45 minutes at 40° C and 1300 rpm. The reaction mixture was cooled to 20°C and 9.0g NaOH-25 was added. The reaction mixture was stirred for 2 minutes. The reaction mixture was quenched with 72.0g demineralized water and stirred for 1 min. Agitation was stopped and the organic phase and water phase were allowed to separate. The water phase was drained from the reactor and discarded.

<u>Reducing agent addition step [Step c)]</u>

To the unstirred reactor content, 3.4g buffer solution (16.3 wt% AcOH, 62.5 wt% water and 21.2 wt% NaOH-25) were added followed by the addition of 100g water. The temperature was kept at 20° C by jacketed temperature control. The pH was adjusted to pH 5.5 by addition of a few drops of NaOH-25. To the buffered solution 56 g of a freshly prepared sulfite solution (69 wt% water, 18 wt% sodium metabisulfite $Na_2S_2O_5$ (s) and 13 wt% NaOH-25) was added dropwise over a period of 6 minutes. During the dosing a pH of 5.5 was maintained by the addition of NaOH-25 (approximately 7g in total).

<u>Example 3 [*step i) - the mixture of step c) has a pH of less than 6.8 before proceeding to step d)*]</u>

After the addition of the sulfite solution, the reaction mixture was stirred for 20 minutes at a pH of 5.5. The pH of the reaction mixture was then increased to 8.0 by the dropwise addition of approximately 1.0 gram NaOH-25 in 5 minutes *(steps d) and e)),* followed by workup (see below).

<u>Example 4 [*step i) - the mixture of step c) has a pH of less than 6.8 before proceeding to step d); and step ii) - after completion of step e) the pH is decreased to a pH of less than 6.8]*</u>

After the addition of the sulfite solution the reaction mixture was stirred for 5 minutes at a pH of 5.5. The pH was then increased to 10 by the dropwise addition of 8.4g NaOH-25 over 5 minutes (*step d)*). The mixture was stirred for 3 minutes at a pH of 10 (*step e)*). The pH was lowered back to 5.5 by the dropwise addition of 5.4g HCl (15 wt% in water) over 5 minutes (*step ii)*). The reaction mixture was stirred for 2 minutes at a pH of 5.5. Total reduction time was 20 minutes (cf. Example 3). The pH of the reaction mixture was then increased to 8.0 by the dropwise addition of approximately 1.0 gram NaOH-25 in 5 minutes, followed by workup (see below).

<u>Workup for Examples 3 and 4:</u>

Agitation was stopped and the organic phase and water phase were allowed to separate. The water phase was

drained from the reactor and discarded. Next, 33.0 gram NaCl-25 (25 wt% in water), 102 gram demineralized water and 36.3 gram $NaHCO_3$-6 (6 wt% $NaHCO_3$ in water) were added to the unstirred reaction mixture. After the addition the reaction mixture was stirred (1000 rpm) at 20° C for 2 minutes. Agitation was stopped and the organic phase and water phase were allowed to separate. The water phase was drained from the reactor and discarded. Subsequently the organic phase was drained and collected separately in a 500 ml Erlenmeyer. The organic phase was dried over 10 grams $MgSO_4.2H_2O$ for 10 minutes. The mixture was filtered over a glass filter and the clear organic peroxide product 246 gram (assay 98.8%) was collected and stored at 4° C.

Analysis: Examples 3 and 4 were analyzed using the same method as is described for Examples 1 and 2 ("TBHP Protocol").

| | TBHP (ppm) t = 0 weeks | TBHP (ppm) t = 2 weeks | TBHP (ppm) t = 4 weeks | ΔTBHP (ppm)* 0-4 weeks |
|---|---|---|---|---|
| Example 3 | 5 | 71 | 105 | 100 |
| Example 4 | 0 | 45 | 75 | 75 |
| | | | | |
| ** A lower Δ TBHP value indicates increased storage stability.* | | | | |

C. Preparation of tert-Butyl peroxybenzoate (CAS: 614-45-9) [Steps a) and b)]

To a 1L jacketed glass reactor, equipped with baffles, a pH electrode, an overhead mechanical stirrer (pitch blade agitator, size 1/3 of the diameter of the reactor) and glycol/water temperature control, 55.9g NaCl-25% (aq.) and 160.0g TBHP (70 wt% in water) were added. The reaction medium was kept at 20° C under agitation (1000 rpm) and 59.9g NaOH-25 solution was added in 5 minutes. After the addition, 57.4g benzoyl chloride was added in 14 minutes during which the temperature was maintained at 20° C. Next, 108.0g benzoyl chloride and 131.9g NaOH-25 solution were added simultaneously over 19 minutes during which the was maintained at 20° C. After the addition the reaction mixture was stirred for an additional 32 minutes at 20° C and 1100 rpm. After the post reaction 20.0g NaOH-25 was added and the mixture was stirred for an additional 5 minutes. Agitation was stopped, and the organic phase and water phase were allowed to separate. The water phase was drained from the reactor and discarded.

Reducing agent addition step [Step c]

*Examples 5 and 6:* To the unstirred reactor content, 45g water and 118g NaCl-25 (aq) were added, and the agitator was restarted (1100 RPM). The pH was adjusted to pH 10 with 6.2g NaOH-25. The temperature was kept at 20° C by jacketed temperature control. To the stirred solution 46g freshly prepared sulfite solution (69 wt% water, 18 wt% sodium metabisulfite $Na_2S_2O_5$ (s) and 13 wt% NaOH-25) was added dropwise over a period of 6 minutes. During the dosing a pH of 10 was maintained by the addition of NaOH-25 (approximately 8.1g in total).

*Examples 7 and 8:* To the unstirred reactor content, 45g water, 118g NaCl-25 (aq.) and 10g buffer solution (16.3 wt% AcOH, 62.5 wt% water and 21.2 wt% NaOH-25) were added. The temperature was kept at 20° C by jacketed temperature control. The pH was adjusted to pH 5.5 by addition of a few drops of NaOH-25. To the buffered solution 46g of a freshly prepared sulfite solution (69 wt% water, 18 wt% sodium metabisulfite $Na_2S_2O_5$ (s) and 13 wt% NaOH-25) was added dropwise over a period of 5 minutes. During the dosing a pH of 5.5 was maintained by the addition of NaOH-25 (approximately 2g in total).

Example 5 *[Comparative]*

The reaction mixture was stirred for an additional 30 minutes after the sulfite solution was dosed and the pH was maintained at pH 10, followed by workup (see below).

Example 6 [*step i) - the mixture of step c) has a pH of less than 6.8 before proceeding to step d*)]l

The reaction mixture was stirred for an additional 25 minutes after the sulfite solution was dosed and the pH was maintained at pH 10. The pH was subsequently lowered to pH 6.4 by the addition of 6.2g HCl (15 wt% in water) in 3 minutes (*step i))*. The reaction mixture was stirred for 2 minutes at pH of 6.4. (total time 30min; cf. Comparative Example 5). The pH was then brough to 10 with the addition of NaOH-25 *(steps d) and e)),* followed by workup (see below).

Example 7 [*step i) - the mixture of step c) has a pH of less than 6.8 before proceeding to step d)*]

After the addition of the sulfite solution, the reaction mixture was stirred for 20 minutes at a pH of 5.5. The pH of the reaction mixture was then increased to 7.6 by the dropwise addition of approximately 5g NaOH-25 in 5 minutes *(steps d) and e)),* followed by workup (see below).

Example 8 [*step i) - the mixture of step c) has a pH of less than 6.8 before proceeding to step d); and step ii) - after completion of step e) the pH is decreased to a pH of less than 6.8]*

After the addition of the sulfite solution the reaction mixture was stirred for 5 minutes at a pH of 5.5. The pH was then increased to 10 by the dropwise addition of 7g NaOH-25 over 5 minutes (*step d)*). The mixture was stirred for 3 minutes at a pH of 10 (*step e)*). The pH was lowered back to 5.5 by the dropwise addition of 9.6g HCl (15 wt% in water) over 5 minutes (*step ii)*). The reaction mixture was stirred for 2 minutes at a pH of 5.5. Total reduction time was 20 minutes (cf. Example 7). The pH of the reaction mixture was then increased to 7.6 by the dropwise addition of approximately 5g NaOH-25 in 5 minutes, followed by workup (see below).

Workup for examples 5-8

Agitation was stopped and the organic phase and water phase were allowed to separate. The water phase was drained from the reactor and discarded. Next, 100g NaCl-25 (25 wt% in water) and 45g demineralized water were added to the unstirred reaction mixture. After the addition the reaction mixture was stirred (1000 rpm) at 20° C for 2 minutes. Agitation was stopped and the organic phase and water phase were allowed to separate. The water phase was drained from the reactor and discarded. Subsequently the organic phase was drained and collected separately in a 500 ml Erlenmeyer. The organic phase was dried over 8g $MgSO_4.2H_2O$ for 10 minutes. The mixture was filtered over a glass filter and the clear organic peroxide product 207 gram (assay 99%) was collected and stored at 20° C.
Analysis: Examples 5-8 were analyzed using the same method as is described for Examples 1 and 2 ("TBHP Protocol").

| | TBHP (ppm) t = 0 weeks | TBHP (ppm) t = 2 weeks | TBHP (ppm) t = 4 weeks | ΔTBHP* (ppm) 0-4 weeks |
|---|---|---|---|---|
| Example 5 *[Comparative]* | 8263 | 9140 | 9350 | 1087 |
| Example 6 | 340 | 1017 | 1037 | 697 |
| Example 7 | 569 | 1068 | 1369 | 800 |
| Example 8 | 16 | 252 | 273 | 257 |

** A lower Δ TBHP value indicates increased storage stability*
***C. Ex. 5** - pH in step c) always >6.8; no pH reduction after completion of step e)*
***Ex. 6** - pH in step c) <6.8 before step d); no pH reduction after completion of step e)*
***Ex. 7** - pH in step c) <6.8 before step d); no pH reduction after completion of step e)*
***Ex. 8** - pH in step c) <6.8 before step d); pH reduction after completion of step e)*

*Conclusions - Examples 1-8*

These data show that having a pH of less than 6.8 in step c) before proceeding to step d) (*"step i*)') reduces the hydroperoxide content in the product (>93% reduction at t=0) and increases storage stability (C.Ex.5 *vs.* Ex. 6/7). These data also demonstrate that reducing the pH following completion of step e) (*"step ii)*") reduces the hydroperoxide content in the product (>86% reduction at t=0) and increases storage stability (Ex. 2 *vs.* Ex. 1; Ex. 4 *vs.* Ex. 3; Ex. 8 vs. Ex. 7).
The overall improvement obtained by combining steps *i)* and *ii)* is remarkable (>99% reduction in hydroperoxide content at t=0, substantial improvement in storage stability; C. Ex. 5 *vs.* Ex. 8). As shown above, this most preferred process (Ex. 2, Ex. 4., Ex. 8) consistently generated t-butylperoxy products with an initial TBHP content of less than 300 ppm TBHP and a 4-week stability (ΔTBHP, 0-4 weeks) of less than 300 ppm.
While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment as contemplated herein. It should be understood that various changes

may be made in the function and arrangement of elements described in an exemplary embodiment without departing from the scope of the invention as set forth in the appended claims.

The present disclosure may be further described by the following Aspects.

**Aspect 1.** A process for preparing a peroxyester or peroxycarbonate comprising:

a) reacting an organic hydroperoxide with an acid halide, an acid anhydride, or a haloformate, in the presence of a base,
b) separating the aqueous layer after completion of step a),
c) adding a reducing agent to the organic layer after the aqueous layer has been separated in step b), wherein the reducing agent is an agent capable of reducing the organic hydroperoxide to the corresponding alcohol,
d) ensuring that the mixture of step c) has a pH of greater than 6.8 by maintaining or increasing the pH of the mixture of step c), and
e) maintaining the pH of greater than 6.8 for at least 5 seconds,

wherein the process is characterized in that:

i. the mixture of step c) has a pH of less than 6.8 before step d), and in step d) the pH of the mixture of step c) is increased to a pH of greater than 6.8; and/or
ii. after completion of step e) the pH is decreased to a pH of less than 6.8.

**Aspect 2.** The process of Aspect 1, wherein the process further comprises increasing the pH to greater than 6.8 and/or washing the organic layer after completion of step ii), wherein the washing step(s) preferably comprise one or more water washes and/or one or more alkaline aqueous washes, such as washing with NaOH(aq) and/or NaHCO3(aq)).

**Aspect 3.** The process of Aspect 1 or 2, wherein the reducing agent added in step c) is a sulfite.

**Aspect 4.** The process of Aspect 3, wherein the sulfite is an aqueous sulfite solution.

**Aspect 5.** The process of any one of Aspects 1-4, wherein in step c) the pH is decreased to a pH of 6.5 or less.

**Aspect 6.** The process of any one of Aspects 1-5, wherein in step c) the pH is decreased to a pH of from about 4 to about 6.5.

**Aspect 7.** The process of any one of Aspects 1-6, wherein step d) comprises ensuring that the pH of the mixture of step c) is >7.0.

**Aspect 8.** The process of any one of Aspects 1-7, wherein step d) comprises ensuring that the pH of the mixture of step c) is >7.2.

**Aspect 9.** The process of any one of Aspects 1-8, wherein step d) comprises ensuring that the pH of the mixture of step c) is >7.4.

**Aspect 10.** The process of any one of Aspects 1-9, wherein step e) the pH is maintained for at least 10 seconds, preferably at least 30 seconds, preferably at least 60 seconds, and more preferably at least 120 seconds.

**Aspect 11.** The process of any one of Aspects 2-10, wherein in step ii) the pH is decreased to a pH of about 6.5 or lower.

**Aspect 12.** The process of any one of Aspects 2-11, wherein in step ii) the pH is decreased to a pH of about 4.5 to 6.5.

**Aspect 13.** The process of any one of Aspects 1-12, wherein the organic hydroperoxide is selected from an organic hydroperoxide of the general formula (II):

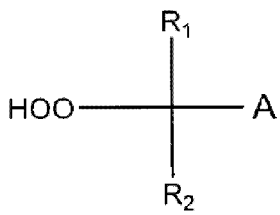

(II)

wherein:

**$R_1$** and **$R_2$** are independently selected from the group comprising hydrogen, $C_1$-$C_{20}$ alkyl, $C_3$-$C_{20}$ cycloalkyl, $C_6$-$C_{20}$ aryl, $C_7$-$C_{20}$ aralkyl, and $C_7$-$C_{20}$ alkaryl, or **$R_1$** and **$R_2$** form a $C_3$-$C_{12}$ cycloakyl group, which groups may include linear or branched alkyl moieties, and each of **$R_1$** and **$R_2$** may optionally be substituted with one or more groups selected from hydroxy, hydroperoxy, alkoxy, linear or branched alkyl, aryloxy, halogen, ester, carboxy, nitrile, and amido,
**A** is selected independently of $R_1$ and $R_2$ from the same group of substituents as $R_1$ and $R_2$, or **A** is of the general

formula (III):

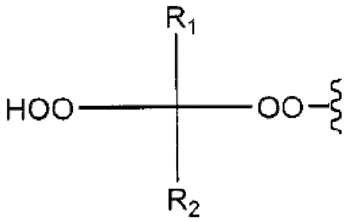

(III)

wherein $\mathbf{R_1}$ and $\mathbf{R_2}$ are as defined above, wherein **A** is preferably selected independently of $R_1$ and $R_2$ from the same group of substituents as $R_1$ and $R_2$.

**Aspect 14.** The process of any one of Aspects 1-13, wherein the organic hydroperoxide is tert-butyl hydroperoxide, tert-amyl hydroperoxide, cumyl hydroperoxide, 1,1,3,3-tetramethylbutyl hydroperoxide, 4-hydroperoxy-4-methylpentan-2-ol, 2,5-dihydroperoxy-2,5-dimethylhex-3-yne, 2,5-dihydroperoxy-2,5-dimethylhexane, or mixtures thereof.

**Aspect 15.** The process of any one of Aspects 1-14, wherein the organic hydroperoxide is tert-butyl hydroperoxide, preferably obtained from an air oxidation process.

**Aspect 16.** The process of any one of Aspects 1 to 15, wherein the acid halide, acid anhydride, or chloroformate is a reactive carbonyl compound of the general formulae (Ia) or (Ib):

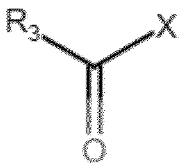

(Ia)

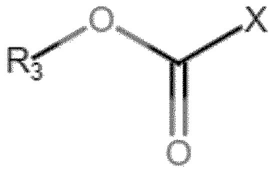

(Ib)

wherein:

$\mathbf{R_3}$ is independently selected from the group comprising $C_1$-$C_{20}$ alkyl, $C_3$-$C_{20}$ cycloalkyl, $C_6$-$C_{20}$ aryl, $C_7$-$C_{20}$ aralkyl, and $C_7$-$C_{20}$ alkaryl, wherein $R_3$ is optionally substituted with one or more groups selected from hydroxy, alkoxy, linear or branched alkyl, aryloxy, halogen, ester, carboxy, nitrile, and amido, and

**X** is a halogen or -O-CO-$R_{3'}$, wherein $R_{3'}$ is selected independently of $R_3$ from the same group of substituents as $R_3$.

**Aspect 17.** The process of any one of Aspects 1 to 16, wherein the acid halide, acid anhydride or haloformate is derived from any of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid, citraconic acid, fumaric acid, oxalic acid, terephthalic acid, propenoic acid, and phthalic acid.

**Aspect 18.** The process of any one of Aspects 1 to 17, wherein the process uses an acid halide or a haloformate.

**Aspect 19.** The process of any one of Aspects 1 to 18, wherein the acid halide is an acid chloride.

**Aspect 20.** The process of Aspect 19, wherein the acyl portion of the acid chloride derives from any of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid, citraconic acid, fumaric acid, oxalic acid, terephthalic acid, propenoic acid, and phthalic acid.

**Aspect 21.** The process of any one of Aspects 1 to 18, wherein the haloformate is a chloroformate.

**Aspect 22.** The process of Aspect 21, wherein the chloroformate is selected from 2-(1-methylethoxy)phenyl

chloroformate, 1-methylpropyl chloroformate, 4-methylphenyl chloroformate, 2,2,2-trichloro-1,1-dimethylethyl chloroformate, heptyl chloroformate, cyclohexyl methyl chloroformate, ethylene glycol bis(chloroformate), 3-(1,1-dimethylethyl) phenyl chloroformate, 3-(trichlorosilyl)propyl chloroformate, phenyl chloroformate, 3-methoxybutyl chloroformate, 2-phenoxyethyl chloroformate, 2,2-dimethyl-1,3-propane diol bis(chloroformate), phenyl methyl chloroformate, 9-octadecenyl chloroformate, 2-methylphenyl chloroformate, bisphenol A bis(chloroformate), 1,3-dimethyl butyl chloroformate, 3,4-dimethyl butyl chloroformate, 3,4-dimethyl phenyl chloroformate, trichloromethyl chloroformate, 1-chloroethyl chloroformate, chloromethyl chloroformate, 1,4-butane diol bis(chloroformate), 1,1-bis (ethoxycarbo)ethyl chloroformate, 3,5-dimethyl phenyl chloroformate, octyl chloroformate, ethyl chloroformate, octadecyl chloroformate, (2-oxo-1,3-dioxolan-4-yl)methyl chloroformate, 1,6-hexane diol bis(chloroformate), 2-chlorobutyl chloroformate, 4-methoxyphenyl chloroformate, 2-methylpropyl chloroformate, 2-(methylsulfonyl)ethyl chloroformate, dodecyl chloroformate, 1,4-cyclohexane dimethanol bis(chloroformate), 2-chloro-2-phenyl ethyl chloroformate, 2-acryloyloxyethyl chloroformate, 4-nitrophenyl chloroformate, n-butyl chloroformate, decyl chloroformate, 2-ethylhexyl chloroformate, 2-propenyl chloroformate, 2-chlorocyclohexyl chloroformate, 2-methyl-2-propenyl chloroformate, cyclohexyl chloroformate, 2-chloroethyl chloroformate, [4-(phenylazo)phenyl]methyl chloroformate, hexadecyl chloroformate, 1-naphthalenyl chloroformate, 2-[2-cyclopentyl-4-(1,1-dimethylethyl)phenoxy]-1-methylethyl chloroformate, 3,5,5-trimethyl hexyl chloroformate, isotridecyl chloroformate, tridecyl chloroformate, 4-(1,1-dimethylethyl)cyclohexyl chloroformate, 2,4,5-trichlorophenyl chloroformate, 3-chloropropyl chloroformate, tetradecyl chloroformate, 9H-fluoren-9-yl methyl chloroformate, (4-nitrophenyl)methyl chloroformate, methyl chloroformate, 2-(1-methylethyl)phenyl chloroformate, triethylene glycol bis(chloroformate), 2-methoxyethyl chloroformate, 1-methylethenyl chloroformate, 3-methylphenyl chloroformate, 2-bromoethyl chloroformate, diethylene glycol bis(chloro-formate), 3-methyl-5-(1-methylethyl)phenyl chloroformate, 2,2,2-tribromoethyl chloroformate, 2-ethoxyethyl chloroformate, 3-methyl-1,5-pentane diol bis(chloroformate), 4-methoxy carbophenyl chloroformate, ethenyl chloroformate, 1-methylethyl chloroformate, 2-(1-methylpropyl)phenyl chloroformate, 2,2,2-trichloroethyl chloroformate, pentyl chloroformate, cyclodecyl chloroformate, 4-(1,1-dimethylethyl)phenyl chloroformate, hexyl chloroformate, n-propyl chloroformate, 3-methoxy-3-methylbutyl chloroformate, 2-propoxyethyl chloroformate, 2-methoxy-1-methylethyl chloroformate, 2-butoxyethyl chloroformate, 2,2-dimethyl propyl chloroformate, 2,3-dihydro-2,2-dimethyl-7-benzofuranyl chloroformate, 1-chloroethyl chloroformate, cyclobutyl chloroformate, 5-methyl-2-(1-methylethyl)cyclohexyl chloroformate, 1,1-dimethyl ethyl chloroformate, 1-methylheptyl chloroformate, and mixtures thereof.

**Aspect 23.** The process of any one of Aspects 1 to 22, wherein the base is selected from sodium carbonate, potassium carbonate, sodium hydroxide, potassium hydroxide, and mixtures thereof.

**Aspect 24.** The process of any one of Aspects 1 to 23, wherein in any one of steps c)-e) and ii) the mixture is agitated for at least 1 minute before proceeding to the next step of the process.

**Aspect 25.** A tert-butyl peroxyester or a tert-butyl peroxycarbonate, characterized in that the tert-butyl peroxyester or tert-butyl peroxycarbonate has an initial (t=0) tert-butylhydroperoxide (TBHP) content of less than 300 ppm and a four-week stability (0-4 week ΔTBHP) value of 300 ppm or less, wherein the initial TBHP content and the four-week stability value are determined in accordance with the "TBHP Protocol".

**Aspect 26.** A tert-butyl peroxyester, characterized in that the tert-butyl peroxyester has an initial (t=0) tert-butylhydroperoxide (TBHP) content of less than 300 ppm and a four-week stability (0-4 week ΔTBHP) value of 300 ppm or less, wherein the initial TBHP content and the four-week stability value are determined in accordance with the "TBHP Protocol".

**Aspect 27.** The tert-butyl peroxyester of Aspect 26, wherein the tert-butyl peroxyester is tert-butyl peroxy-3,5,5-trimethyl hexanoate (CAS: 13122-18-4), tert-Butyl peroxy-2-ethylhexanoate (CAS: 3006-82-4), or tert-Butyl peroxybenzoate (CAS: 614-45-9).

## Claims

**1.** A process for preparing a tert-butyl peroxyester or a tert-butyl peroxycarbonate comprising:

a) reacting tert-butyl hydroperoxide with an acid halide, an acid anhydride, or a haloformate, in the presence of a base,
b) separating the aqueous layer after completion of step a),
c) adding a reducing agent to the organic layer after the aqueous layer has been separated in step b), wherein the reducing agent is an agent capable of reducing tert-butyl hydroperoxide to tert-butyl alcohol,
d) ensuring that the mixture of step c) has a pH of greater than 6.8 by maintaining or increasing the pH of the mixture of step c), and
e) maintaining the pH of greater than 6.8 for at least 5 seconds,

wherein the process is **characterized in that**:

i. the mixture of step c) has a pH of less than 6.8 before step d), and in step d) the pH of the mixture of step c) is increased to a pH of greater than 6.8; and/or

ii. after completion of step e) the pH is decreased to a pH of less than 6.8.

**2.** The process of Claim 1, wherein the reducing agent added in step c) is a sulfite.

**3.** The process of Claim 2, wherein the sulfite is an aqueous sulfite solution.

**4.** The process of any one of Claims 1-3, wherein in step i) the mixture of step c) has a pH of 6.5 or less.

**5.** The process of any one of Claims 1-4, wherein in step i) the mixture of step c) has a pH of from about 4 to about 6.5.

**6.** The process of any one of Claims 1-5, wherein step d) comprises ensuring that the pH of the mixture of step c) is greater than 7.0.

**7.** The process of any one of Claims 1-6, wherein in step ii) the pH is decreased to a pH of about 6.5 or lower.

**8.** The process of any one of Claims 1-7, wherein the tert-butyl hydroperoxide is obtained from an air oxidation process.

**9.** The process of any one of Claims 1-8, wherein the acid halide, acid anhydride, or haloformate is a reactive carbonyl compound of the general formulae (Ia) or (Ib):

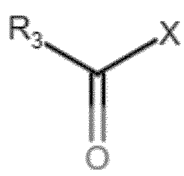
(Ia)

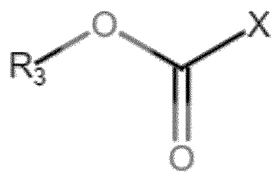
(Ib)

wherein:

**$R_3$** is independently selected from the group comprising $C_1$-$C_{20}$ alkyl, $C_3$-$C_{20}$ cycloalkyl, $C_6$-$C_{20}$ aryl, $C_7$-$C_{20}$ aralkyl, and $C_7$-$C_{20}$ alkaryl, wherein $R_3$ is optionally substituted with one or more groups selected from hydroxy, alkoxy, linear or branched alkyl, aryloxy, halogen, ester, carboxy, nitrile, and amido, and

**X** is a halogen or -O-CO-$R_{3'}$, wherein $R_{3'}$ is selected independently of $R_3$ from the same group of substituents as $R_3$.

**10.** The process of any one of Claims 1-9, wherein the acid halide, acid anhydride or haloformate is derived from any of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, 3,5,5-trimethylpentanedioic acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid, citraconic acid, fumaric acid, oxalic acid, terephthalic acid, propenoic acid, and phthalic acid.

**11.** The process of any one of Claims 1-10, wherein the process uses an acid halide or a haloformate, preferably an acid chloride or a chloroformate.

**12.** The process of Claim 11, wherein the acyl portion of the acid chloride corresponds to the acyl portion of any one of the following carboxylic acids: acetic acid, phenylacetic acid, phenoxyacetic acid, propanoic acid, isobutyric acid, n-butyric acid, benzoic acid, 2-methyl-benzoic acid, 2-methylbutanoic acid, 2-butenoic acid, 3-phenylpropenic acid, 2,2-dimethylpropanoic acid, 2,2-dimethylbutanoic acid, 2,2-dimethylpentanoic acid, 2-ethylbutanoic acid, 3,5,5-trimethylhexanoic acid, 2-ethylhexanoic acid, neohexanoic acid, neoheptanoic acid, neodecanoic acid, octanoic acid, nonanoic acid, lauric acid, 3,5,5-trimethylpentanedioic acid, hexanedioic acid, 3,5,5-trimethylhexanedioic acid, 2,4,4-trimethylhexanedioic acid, decanedioic acid, undecanedioic acid, dodecanedioic acid, cyclohexanecarboxylic acid, 1,4-cyclohexanedicarboxylic acid, cyclohexane-1,4-diacetic acid, maleic acid, citric acid, methylsuccinic acid,

citraconic acid, fumaric acid, oxalic acid, terephthalic acid, propenoic acid, and phthalic acid; and/or wherein the chloroformate is selected from 2-(1-methylethoxy)phenyl chloroformate, 1-methylpropyl chloroformate, 4-methylphenyl chloroformate, 2,2,2-trichloro-1,1-dimethylethyl chloroformate, heptyl chloroformate, cyclohexyl methyl chloroformate, ethylene glycol bis(chloroformate), 3-(1,1-dimethylethyl)phenyl chloroformate, 3-(trichlorosilyl)propyl chloroformate, phenyl chloroformate, 3-methoxybutyl chloroformate, 2-phenoxyethyl chloroformate, 2,2-dimethyl-1,3-propane diol bis(chloroformate), phenyl methyl chloroformate, 9-octadecenyl chloroformate, 2-methylphenyl chloroformate, bisphenol A bis(chloroformate), 1,3-dimethyl butyl chloroformate, 3,4-dimethyl butyl chloroformate, 3,4-dimethyl phenyl chloroformate, trichloromethyl chloroformate, 1-chloroethyl chloroformate, chloromethyl chloroformate, 1,4-butane diol bis(chloroformate), 1,1-bis (ethoxycarbo)ethyl chloroformate, 3,5-dimethyl phenyl chloroformate, octyl chloroformate, ethyl chloroformate, octadecyl chloroformate, (2-oxo-1,3-dioxolan-4-yl) methyl chloroformate, 1,6-hexane diol bis(chloroformate), 2-chlorobutyl chloroformate, 4-methoxyphenyl chloroformate, 2-methylpropyl chloroformate, 2-(methylsulfonyl)ethyl chloroformate, dodecyl chloroformate, 1,4-cyclohexane dimethanol bis(chloroformate), 2-chloro-2-phenyl ethyl chloroformate, 2-acryloyloxyethyl chloroformate, 4-nitrophenyl chloroformate, n-butyl chloroformate, decyl chloroformate, 2-ethylhexyl chloroformate, 2-propenyl chloroformate, 2-chlorocyclohexyl chloroformate, 2-methyl-2-propenyl chloroformate, cyclohexyl chloroformate, 2-chloroethyl chloroformate, [4-(phenylazo)phenyl]methyl chloroformate, hexadecyl chloroformate, 1-naphthalenyl chloroformate, 2-[2-cyclopentyl-4-(1,1-dimethylethyl)phenoxy]-1-methylethyl chloroformate, 3,5,5-trimethyl hexyl chloroformate, isotridecyl chloroformate, tridecyl chloroformate, 4-(1,1-dimethylethyl)cyclohexyl chloroformate, 2,4,5-trichlorophenyl chloroformate, 3-chloropropyl chloroformate, tetradecyl chloroformate, 9H-fluoren-9-yl methyl chloroformate, (4-nitrophenyl)methyl chloroformate, methyl chloroformate, 2-(1-methylethyl)phenyl chloroformate, triethylene glycol bis(chloroformate), 2-methoxyethyl chloroformate, 1-methylethenyl chloroformate, 3-methylphenyl chloroformate, 2-bromoethyl chloroformate, diethylene glycol bis(chloro-formate), 3-methyl-5-(1-methylethyl)phenyl chloroformate, 2,2,2-tribromoethyl chloroformate, 2-ethoxyethyl chloroformate, 3-methyl-1,5-pentane diol bis(chloroformate), 4-methoxy carbophenyl chloroformate, ethenyl chloroformate, 1-methylethyl chloroformate, 2-(1-methylpropyl)phenyl chloroformate, 2,2,2-trichloroethyl chloroformate, pentyl chloroformate, cyclodecyl chloroformate, 4-(1,1-dimethylethyl)phenyl chloroformate, hexyl chloroformate, n-propyl chloroformate, 3-methoxy-3-methylbutyl chloroformate, 2-propoxyethyl chloroformate, 2-methoxy-1-methylethyl chloroformate, 2-butoxyethyl chloroformate, 2,2-dimethyl propyl chloroformate, 2,3-dihydro-2,2-dimethyl-7-benzofuranyl chloroformate, 1-chloroethyl chloroformate, cyclobutyl chloroformate, 5-methyl-2-(1-methylethyl)cyclohexyl chloroformate, 1,1-dimethyl ethyl chloroformate, 1-methylheptyl chloroformate, and mixtures thereof.

**13.** A tert-butyl peroxyester or a tert-butyl peroxycarbonate, **characterized in that** the tert-butyl peroxyester or tert-butyl peroxycarbonate has an initial (t=0) tert-butylhydroperoxide (TBHP) content of less than 300 ppm and a four-week stability (0-4 week ΔTBHP) value of 300 ppm or less, wherein the initial TBHP content and the four-week stability value are determined in accordance with the "TBHP Protocol".

**14.** A tert-butyl peroxyester, **characterized in that** the tert-butyl peroxyester has an initial (t=0) tert-butylhydroperoxide (TBHP) content of less than 300 ppm and a four-week stability (0-4 week ΔTBHP) value of 300 ppm or less, wherein the initial TBHP content and the four-week stability value are determined in accordance with the "TBHP Protocol".

**15.** The tert-butyl peroxyester of claim 14, wherein the tert-butyl peroxyester is tert-butyl peroxy-3,5,5-trimethyl hexanoate (CAS: 13122-18-4), tert-Butyl peroxy-2-ethylhexanoate (CAS: 3006-82-4), or tert-Butyl peroxybenzoate (CAS: 614-45-9).

Europäisches Patentamt
European Patent Office
Office européen des brevets

# EUROPEAN SEARCH REPORT

Application Number
EP 25 17 2444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 300 044 A (GUANGDONG ZHONGZHUN NEW MATERIAL TECH CO LTD) 2 February 2021 (2021-02-02) | 13-15 | INV. C07C409/38 C07C407/00 |
| A | * claim 1; examples 1-4 * | 1-12 | |
| X | EP 1 382 596 A1 (ATOFINA CHEM INC [US]) 21 January 2004 (2004-01-21) | 13-15 | |
| A | * examples 1-13 * | 1-12 | |
| X | KRASUTSKY PAVEL A. ET AL: "Double- and Triple-Consecutive O-Insertion into tert -Butyl and Triarylmethyl Structures", ORGANIC LETTERS, vol. 6, no. 15, 29 June 2004 (2004-06-29), pages 2539-2542, XP093215744, US ISSN: 1523-7060, DOI: 10.1021/ol049171p | 13-15 | |
| A | * scheme 2 * | 1-12 | |
| | | | TECHNICAL FIELDS SEARCHED (IPC) |
| | | | C07C |

The present search report has been drawn up for all claims

1

| Place of search | Date of completion of the search | Examiner |
|---|---|---|
| The Hague | 2 September 2025 | Matés Valdivielso, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 17 2444

This annex lists the patent family members relating to the patent documents cited in the above-mentioned European search report. The members are as contained in the European Patent Office EDP file on
The European Patent Office is in no way liable for these particulars which are merely given for the purpose of information.

02-09-2025

| Patent document cited in search report | Publication date | Patent family member(s) | Publication date |
|---|---|---|---|
| CN 112300044 A | 02-02-2021 | NONE | |
| EP 1382596 A1 | 21-01-2004 | AT E252556 T1 | 15-11-2003 |
| | | AT E353873 T1 | 15-03-2007 |
| | | AU 719016 B2 | 04-05-2000 |
| | | BR 9706638 A | 12-01-1999 |
| | | CA 2232361 A1 | 26-02-1998 |
| | | CN 1198734 A | 11-11-1998 |
| | | DE 69725708 T2 | 29-07-2004 |
| | | DE 69737374 T2 | 23-08-2007 |
| | | EP 0879224 A1 | 25-11-1998 |
| | | EP 1382596 A1 | 21-01-2004 |
| | | HK 1016162 A1 | 29-10-1999 |
| | | JP 4368422 B2 | 18-11-2009 |
| | | JP 5079751 B2 | 21-11-2012 |
| | | JP 2002514172 A | 14-05-2002 |
| | | JP 2009280828 A | 03-12-2009 |
| | | MX PA98003195 A | 11-09-2003 |
| | | MY 115470 A | 30-06-2003 |
| | | PL 326968 A1 | 09-11-1998 |
| | | RU 2194695 C2 | 20-12-2002 |
| | | US 5760149 A | 02-06-1998 |
| | | WO 9807684 A1 | 26-02-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1382596 A
- CN 112300044


### Non-patent literature cited in the description


- *CHEMICAL ABSTRACTS*, 13122-18-4
- *CHEMICAL ABSTRACTS*, 3006-82-4
- *CHEMICAL ABSTRACTS*, 614-45-9